# EUROPEAN PATENT APPLICATION

(11) **EP 4 625 361 A1**
(43) Date of publication of application: **01.10.2025**
(21) Application number: 24197229.8
(22) Date of filing: 29.08.2024
(51) Int. Cl.: G06V 20/52, G06V 10/98, G06V 10/25, H04N 7/18, G06V 40/10

(54) **INFANT MONITORING**

(30) Priority: 28.03.2024 EP 24167699
(71) Applicant: Koninklijke Philips N.V., 5656 AG Eindhoven (NL)
(72) Inventor: GOSENSHUIS, Daan Hendrik, Eindhoven (NL); BERNTSEN, Luc, Eindhoven (NL)
(74) Representative: Philips Intellectual Property & Standards

(57) **Abstract**

Proposed concepts aim to provide schemes, solutions, concepts, designs, methods, and systems pertaining to determining a region of interest for use in monitoring an infant. In particular, embodiments aim to provide a method for determining a region of interest for use in monitoring an infant by processing an image of an area using a feature detection model to detect a feature of the infant's body. Based on the position of the detected feature within the area, a region of interest within the area is then determined. Such a region of interest may help to identify image data than can be ignored or deprioritized by infant one or more monitoring processes, thus reducing an amount of processing/analysis required to be undertaken by the process(es).

## Description

### FIELD OF THE INVENTION

This invention relates to the field of infant monitoring, and in particular to image-based infant sleep monitoring.

### BACKGROUND OF THE INVENTION

Image-based infant monitoring systems are often used by parents and carers to monitor an infant whilst they sleep, allowing the parent/carer to check in on the sleeping infant remotely. Infant monitoring systems are often provided with one or more sensors to take visual and/or audio data of an area (i.e. a monitored area) which may then be relayed to the parent via a separate device or via an app on the parent's smart phone or other personal smart device.

Modern image-based infant monitoring system may provide various functionalities for tracking and monitoring the behaviour of an infant, such as a sleeping infant. These rely on processing image data of the infant or their bed/crib to extract information regarding the infant, for example the movement and vital signs of the infant. This may be achieved by processing the image data with a variety of different models and algorithms including motion detection models, pixel shift algorithms, object detection models, and edge detections models. In order that the output of these processing techniques is accurate, very expensive technology (radar/Lidar) and/or high computational power is typically required.

### SUMMARY OF THE INVENTION

The invention is defined by the claims.

According to an aspect of the invention, there is provided a method for determining a region of interest for use in monitoring an infant within an area. The method comprises: processing first image data of the area with a feature detection model to detect a feature of the infant's body; and determining, based on the position of the detected feature within the area, a region of interest within the area, wherein the region of interest is a region to be used for monitoring the infant.

Such a method may help to identify a reduced portion of the image data (i.e. a subsection of a captured image) that may be used for infant monitoring, thus enabling robust and/or accurate monitoring whilst reducing computational/processing requirements.

Proposed concepts thus aim to provide schemes, solutions, concepts, designs, methods, and systems pertaining to determining a region of interest for use in monitoring an infant. In particular, embodiments aim to provide a method for determining a region of interest for use in monitoring an infant by processing an image of an area using a feature detection model to detect a feature of the infant's body. Based on the position of the detected feature within the area, a region of interest within the area is then determined. Such a region of interest may help to identify image data than can be ignored or deprioritized by infant one or more monitoring processes, thus reducing an amount of processing/analysis required to be undertaken by the process(es).

Further, in some embodiments, the region of interest may be modified or adapted if a change in the feature is detected in subsequently captured image data. That is, the region of interest may be changed/updated according to changes in the position and/or orientation of the feature over time, for example.

Infant monitoring preferably takes place during infant sleep, and it may provide information about the infant's sleep (such as periods of sleep and periods when awake). However, the proposed concept(s) may be used more generally for image-based vital signs monitoring or other image-based monitoring functions, for example whether the infant is awake or asleep, or in light or deep sleep.

Embodiments are described below, simply as one or more preferred implementations of the invention, with reference to infant breathing and/or sleep monitoring.

It has been realised that one way of ensuring accurate image-based monitoring of an infant, such as a sleeping infant, is to crop image data taken of a monitored area (i.e. field of view of the image capture device) to include only a region of interest that contains the image data to be used for monitoring the infant. For example, an image of an entire infant's crib may be cropped to only include an area of the mattress where the infant is determined to be located. This may remove or reduce noise produced by the background of the image and reduce processing requirements. The proposed method leverages feature detection to determine the boundary of this cropped region (i.e. the region of interest). That is, embodiments make use of feature detection to locate a region of interest within image data corresponding to a field of view of the image capture device. This may, for example, support improved breathing and/or sleep monitoring by reducing an amount of data to be processed/analysed by monitoring process.

The inventors propose that only a sub-selection of a captured image may be analysed for infant monitoring whilst maintaining (or even improving) monitoring accuracy, by determining the sub-selection based on automatic feature detection. This may reduce a required computational power and may also reduce a chance of false positive movement artifacts.

Ultimately, an improved computer-implemented method for determining a region of interest for use in monitoring an infant may be supported by the proposed concept(s).

In particular, it is proposed that a single input image of an infant laying in an area may be analyzed, in order to obtain an identification of the infant's position/location within the area. The identified position/location of the infant may then be used to define a region of interest within the image of the area, wherein the region of interest is a sub-region (i.e. a cropped portion) of the image. By restricting subsequent processing or analysis to image data of the region of interest, non-relevant image data may then be excluded from use and thus reduce processing/analysis requirements.

Such an input image may be obtained by a device that a user may commonly already have access to, such as a (camera-based) infant monitor, webcam, or smartphone. Also, by only needing a single input image (i.e. first image data), embodiments may support identification of an infant's location or position without any specialized hardware, e.g. based on images from a camera-based infant monitor, webcam, or smartphone. An improved method for monitoring the breathing (or other physiological process or sign) of a laying infant may therefore be provided by proposed concepts.

Ultimately, an improved method for detecting monitoring an infant within a monitored by an image capture device may be supported by the proposed concept(s).

In some embodiments, the method may further comprise: determining a body axis based on at least one of the detected feature and the first image data, wherein the body axis describes the longitudinal direction of the upper torso of the infant, and the step of determining a region of interest within the area may then be further based on the determined body axis. For instance, by automatically processing information about the detected features and/or the first image data in order to determine the longitudinal direction of the upper torso of the infant (i.e. body axis), a prediction of the infant's body direction and/or posture may be determined (e.g. by analyzing the orientation of one or more detected features). That is, it is proposed that determination of the infant/s laying direction and/or posture may be leveraged to further determine and define the region of interest. Such a proposed approach may be simple but robust to the positioning of the image capture device. For instance, the use of orientation of an infant's shoulder(s) relative to the infant's body axis in captured image (or video) may permit, for instance, simple identification of the infant's laying posture without the use of any special hardware, i.e., with only a conventional video camera, webcam, or smartphone. A location, shape and/or size of the region of interest may then be determined so that it closely surrounds the infant's body.

Also, determining a region of interest within the area may comprise determining a measure of at least one of a position, an orientation, size and shape of the region of interest based on at least one of the detected feature and the determined body axis. For instance, the measure of position may comprise at least one of: an offset magnitude; and direction. This may enable the region of interest to be suitably positioned in consideration of the body axis (e.g. to ensure that the region of interest includes the infant's torso in addition to the infant's head). Further, the measure of the orientation of the region of interest may comprise at least one of: portrait with respect to a horizontal axis of the area; landscape with respect to a horizontal axis of the area; and an angle between an axis of the region of interest and the horizontal axis of the area. The orientation of the region of interest may be a useful metric to ensure that the region of interest is suitably orientated for infant monitoring.

In some embodiments, the measure of the size of the region of interest may comprises at least one of: an area of the region of interest; a dimension of the region of interest; and the proportion of the area covered by the region of interest. Thus, any suitable measurement of the region of interest may be used as a measure of its size. Different measures may be useful for different infant monitoring functions, for example.

In some embodiments, determining a region of interest may comprise determining at least one of: a boundary; a shape; and a validity of the region of interest. By way of further example, determining at least one of: a boundary; shape; and a size of the region of interest may comprise processing the first image data with at least one of: an object detection model; an edge detection model; and a contrast enhancement model. For example, in the case wherein the area corresponds to the infant's crib, an object or feature detection model may be trained to detect an area of an image that depicts an outline of the infant's body in the crib and define the bounding box associated with this detection. In another example, an object or feature detection model may be trained to detect a mattress area or crib area which, in turn, may be employed in determining the region of interest (e.g. the region of interest may be refined or qualified/confirmed based on the position of a detected mattress area relative to a position of the detected infant's head). This may allow for the process of determining, refining and/or checking accuracy of the region of interest to be entirely automated.

In an exemplary embodiment, the feature model may be trained to detect at least one body part of the infant. For instance, the at least one body part of the infant may comprise at least one of: an eye; a nose; an ear; a mouth; a head; a shoulder; a torso; an arm; a leg; a knee; an ankle; a hand; and a foot. In this way, the object detection model determines a location or position of the infant in the first image data by identifying the presence of infant body parts in the image data. The body part may, for example, be the head of the infant as this is the body part most usually uncovered by blankets and therefore detection of an infant head may be a most reliable metric for detecting infant presence and location.

In some embodiments, the method may further comprise: processing second image data of the area with the feature detection model to detect the feature of the infant's body in the second image data, wherein first image data is associated with a first image capture time, and wherein the second image data is associated with a second image capture time subsequent to the first image capture time; and determining, based on the position of the feature detected in the second image data, an adjustment to the region of interest.

In some embodiments, the first image data and second image data of the monitored target area may comprise a plurality of time-sequenced images. This allows for the detection of movement that occurs in the time intervals between each of the time-sequenced images by detecting changes in the image data over time.

Also, the first image data and the second image data may in some embodiments be from a single image sensor, thereby removing the need to have multiple sensors monitoring the target area.

Further, determining an adjustment to the region of interest may comprise: determining a measure position of the feature detected in the second image data relative to a predetermined reference; determining the region of interest is to be adjusted if the measure of position is meets a predetermined condition; and determining the region of interest is not to be adjusted if the measure of position does not meet the predetermined condition. Thus, the method may allow for a simple binary determination of whether region of interest is to be adjusted based on a predetermined threshold value. This may allow for known factors about the required change of feature position to be incorporated into the region of interest adjustment assessment step. In this way, predefined ranges may be provided to define the logic of the adjustment determination.

For instance, the predetermined reference may be based on at least one of: the position of the feature detected in the first image data; the centre of the region of interest; and an edge of the region of interest. Also, the predetermined condition may require that the measure of position exceeds a predetermined threshold value. A determination that region of interest is to be adjusted may thus require a change in position of the detected feature over time period to exceed a certain amount (and the certain amount may be an absolute amount or defined relative to the position/size/area of the region of interest).

In some embodiments, monitoring the infant may comprise at least one of: monitoring one or more of the infant's vital signs; monitoring the infant's movement; monitoring the infant's position; and tracking the infant's sleep stages. For example, the infant's vital signs monitored may include at least one of: body temperature; pulse rate; respiration rate; and blood pressure. Image-based monitoring of a sleeping infant may track various behaviours of the infant to gather information about the quantity and quality of sleep the infant is getting. These different monitoring functionalities may have different requirements regarding the aspect and/or part of the infant to be monitored. Therefore, the region of interest determination method of the present invention may be tailored depending on specific subsequent processing steps intending to be carried out on the image.

In some embodiments, the method may further comprise segmenting the region of interest into a plurality of sub-regions for use in a pixel shift analysis algorithm. Splitting the region of interest into multiple blocks may support more robust analysis by catering for infant movement in different directions.

Some embodiments may further comprise determining the region of interest is to be rejected (i.e. the region of interest is invalid) if it does not meet a predetermined requirement. Such a predetermined requirement may, for example, requires that the region of interest is within a predetermined region of the area. In this way, the validity or relevance of a region of interest may be checked, thus helping to reduce or avoid false positives. Embodiments may therefore cater for the mistaken detection of objects that may accompany a laying infant, such as toys.

In some embodiments, there is provided a computer program comprising code means for implementing any of the methods described above when said program is run on a processing system.

Further, in an exemplary embodiment, there is provided a computer program comprising code means for invoking or executing a computer program that, in turn comprises code means for implementing a method according to a proposed embodiment. That is, a computer program may be provided for triggering the execution of a method according to a proposed embodiment. By way of example, a computer program may be provided to trigger, from a first processing arrangement (e.g. on-premise), the execution of a program, in a second processing arrangement (e.g. off-premise) for implementing a proposed method.

According to another aspect of the invention, there is provided a processor arrangement configured to run a computer program according to a proposed embodiment.

Further, in an exemplary embodiment, there is provided a first processor arrangement configured to control a second, different processor arrangement, the second processor arrangement being configured to implement a method according to a proposed embodiment. That is, a first processor arrangement (e.g. local computing device) may be provided for controlling the execution of a proposed method by a second processor arrangement (e.g. cloud-based execution environment).

According to another aspect of the invention, there is provided a system for determining a region of interest of an area for use in monitoring an infant within the area, the system comprising a processing arrangement configured to run the computer program described above.

In some embodiments, the system or processor arrangement described above may further comprise an output interface configured to receive, from the system or processor arrangement, and display an indication of the determined region of interest.

The system may be remotely located from a user device for monitoring an infant. In this way, a user (such as a parent or carer) may have an appropriately arranged system that can receive information at a location remotely located from the system for determining a region of interest within an area monitored by a camera. Embodiments may therefore enable a user to monitor the infant using a local system (which may, for example, comprise a portable display device, such as a laptop, tablet computer, mobile phone, PDA, etc.). By way of example, embodiments may provide an application for a mobile computing device, and the application may be executed and/or controlled by a user of the mobile computing device.

The system may further include: a server device comprising the system for determining a region of interest of an area within an area monitored by a camera (the area corresponding to the field of view of the camera, for example); and a client device comprising an output-interface. Dedicated data processing means may therefore be employed for the purpose of determining a region of interest within the area monitored by a camera, thus enabling image data processing to be focused or restricted to the region of interest and, in turn, reducing processing requirements or capabilities of other components or devices of the system.

The system may further include a client device, wherein the client device comprises the processing arrangement and a display unit. In other words, a user (such as a parent or carer) may have an appropriately arranged client device (such as a laptop, tablet computer, mobile phone, PDA, etc.) which processes received image data in order to determine a region of interest within a monitored area (e.g. capture area or field of view of an image capture device). Purely by way of example, embodiments may therefore provide a monitoring or observation system that enables targeted monitoring (i.e. monitoring of only a region of interest within the capture area or field of view) of one or more infants from a single location, wherein real-time communication between an infant and monitoring user (e.g. parent or carer) is provided and can have its functionality extended or modified according to proposed concepts, for example.

It will be understood that processing capabilities may therefore be distributed throughout the system in different ways according to predetermined constraints and/or availability of processing resources.

Thus, another aspect of the invention may provide an infant monitoring system that comprises: a system for determining a region of interest of an area for use in monitoring an infant within the area according to a proposed embodiment; an image sensor configured to capture image data of the infant in the area; and an output interface configured to output an indication of the determined region of interest. In this way the image sensor and the system or processing arrangement used to process the image data may be present in a single device along with an interface that displays to the user both the area captured by the image sensor and the determined region of interest used for monitoring the infant. This region of interest may, for example, correspond to a small region (relative to the size of the area captured by the image sensor) surrounding the head and/or torso of the infant and, in this way, the system may be configured to only process image data of the region of interest (and not image data of the entire area) in order to monitor the infant, thereby reducing processing needs/requirements of the infant monitoring system.

In some embodiments, the processing arrangement of the infant monitoring system may be configured to provide instructions to automatically adjust the operating parameters of the image sensor. Thus, the system may be equipped with the ability to automatically adjust the set-up of the image sensor to ensure that the region of interest is sized and/or positioned in the field of view of the image sensor according to predetermined requirements, removing a need for user input and thereby reducing instances of user produced errors to the image sensor arrangement.

Embodiments may be employed in combination with conventional/existing infant monitoring methods and systems. In this way, embodiments may integrate into legacy systems to improve and/or extend their functionality and capabilities. An improved infant monitoring system may therefore be provided by proposed embodiments.

Thus, there may be proposed concepts for determining a region of interest for use in monitoring an infant within an area, and this may be done based on processing an image of the area with a feature detection model and basing the region of interest on the position of a detected feature within the area. These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 depicts a simplified flow diagram of a method for determining a region of interest for use in monitoring an infant within an area according to a proposed embodiment;
Fig. 2 illustrates an exemplary region of interest determined according to the method of Fig. 1;
Fig. 3 is a simplified flow diagram of a method for determining a region of interest according to another embodiment; and
Figs. 4A-4D illustrate exemplary regions of interest determined according to the method of Fig. 3;
Fig. 5 depicts a simplified flow diagram of a method for determining a region of interest according to yet another embodiment;
Fig. 6A-6C illustrate an exemplary implementation of the method of Fig. 5;
Fig. 7 depicted a simplified flow diagram of a method for determining the breathing status of an infant in a monitored area according to a proposed embodiment;
Fig. 8 illustrates an exemplary region of interest determined according to another embodiment of the invention;
Fig. 9A-9D illustrate an exemplary implementation of determining a region of interest according to another embodiment;
Fig. 10 is simplified block diagram of an infant monitoring system for use in monitoring an infant within an area according to a proposed embodiment; and
Fig. 11 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art of practicing the claimed invention, from a study of the drawings, the disclosure, and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to determining a region of interest for use in monitoring an infant situated within a monitored area (for example, the mattress area of a cot/crib being monitored by a video infant monitor). According to proposed concepts, several possible solutions may be implemented separately or jointly. That is, although these possible solutions may be described below separately, two or more of these possible solutions may be implemented in one combination or another.

Embodiments of the invention aim to provide a concept for automatically determining a sub-region of area in which an infant is situated for monitoring. This can be achieved by automatically processing a captured image (e.g. frame of a captured video) of the area. In particular, it is proposed to process first image data of the area with a feature detection model to detect a feature of the infant's body, and then to determine, based on the position of the detected feature within the area, a region of interest within the area. The region of interest is then proposed as a region to be used for monitoring the infant. That is, it is proposed to identify and locate a feature of the infant's body to determine a sub-portion of the image data (i.e. a region of interest) that may be preferred for monitoring the infant. Such a proposed approach may enable a reduction to a false or inaccurate monitoring by identifying relevant/important image data (e.g. within the region of interest) and, in turn, enabling the identification of irrelevant/extraneous image data (e.g. outside of the region of interest).

The image data used in the disclosed method may be in any appropriate form. This may include full or partial images, or full or partial frames of video data. The image data may be obtained using any suitable image sensor or camera-equipped device. For example, the first and second image data may comprise a first and second cropped frames of video data from a video-based infant monitor. The cropped area may be predefined or alternatively may be selected by the user.

The proposed approach may have the additional advantage that it can be carried out using a single image sensor which may form part of any commonly-used device that has image/video capture capabilities. For example, this method could be implemented using data from known infant monitors, smartphone devices, or webcams. Embodiments may therefore leverage and/or employ existing monitoring equipment. Embodiments may also be implemented to extend the functionalities of conventional infant monitoring systems.

Proposed embodiments thus aim to provide an indication of a sub-region of image data that is pertinent/relevant for one or more infant monitoring processes. This image data within the sub-region (i.e. the region of interest) may then be used in an automatic monitoring process of an infant monitoring system, whereas image data outside of the sub-region may be de-prioritized, discarded or ignored. Accordingly, proposed embodiments may provide one or more of the following advantages:
- Do not require advanced processor - may run on single core processor;
- Do not require processing in the cloud;
- Do not require the use of a wearable;
- Accurate breathing rate and motion/breathing rate driven sleep tracking; and
- Eliminates potential disturbances.

Also, video data from an image sensor of the image monitoring system may be sampled and processed at regular time intervals, for example every second, to repeat the region feature detection processes. The region of interest may then be adjusted to cater for any changes in location of the detected feature over time. Such time-sequential detections of an infant's feature may thus be used dynamically and automatically adjust the region of interest according to movement of the infant within the area.

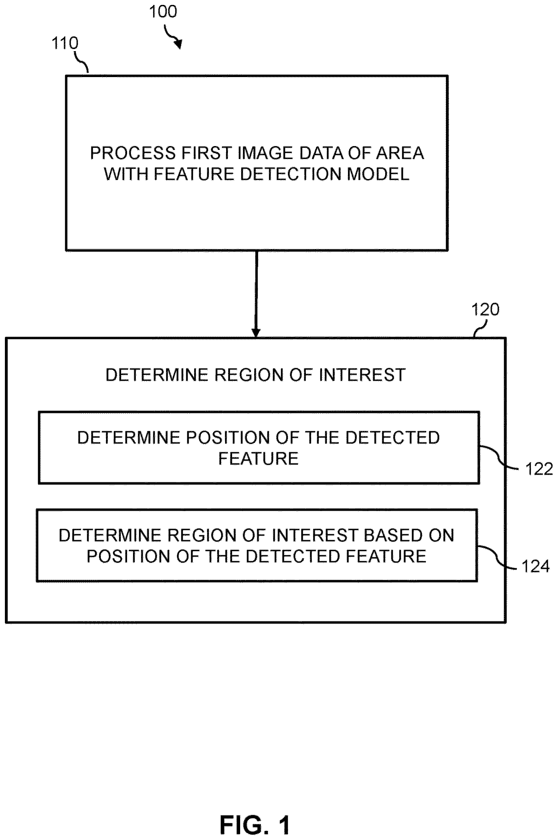

Referring now to Fig. 1, there is depicted a simplified flow diagram of a method 100 for determining a region of interest for use in monitoring an infant within an area. Here, monitoring the infant comprises monitoring the infant's breathing, but in other embodiments is may comprise: monitoring the infant's vital signs; monitoring the infant's movement; monitoring the infant's position; and/or tracking the infant's sleep stages.

The method begins with a step 110, comprising processing first image data of the area with a feature detection model to detect a feature of the infant's body. The first image data, in this embodiment, is a frame of a video taken by a video baby monitor. Also, the feature detection model in this exemplary embodiment is an AI model trained to detect the presence of an infant's head in the image of the area. Any well-known suitable object detection model may be used, for example YOLO, R-CNN, Mask R-CNN, MobileNet, or Squeeze Det. The feature detection model may be a deep learning model trained on a large data set of annotated images to identify features in an image, and classify each feature.

Once the feature of the infant (i.e. the infant's head) has been detected, the method proceeds to a step 120. Step 120 comprises determining, based on the position of the detected feature within the area, a region of interest within the area. This region of interest is a region to be used for monitoring the infant.

In this exemplary embodiment, step 120 comprises the sub-steps 122 and 124. Step 122 comprises determining the position of the detected feature within the area, and step 124 comprises determining the region of interest based on the position of the detected feature (as determined in step 122). Therefore, the sub-steps 122 and 124 involve identifying a position of the feature withing the area and then determining the region according to the position of the feature.

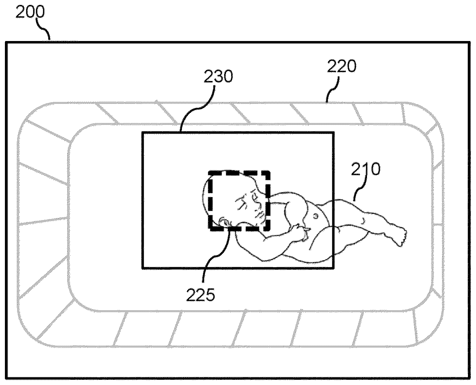

For example, as illustrated in Figure 2, the area 200 in which an infant 210 is to be monitored corresponds to a field of view of the video baby monitor (and thus the field of view of first image data). Within the field of view (and thus within the field of view of first image data) is a cot 220 within which the infant 210 is laying. By processing the first image data according to step 110 the infant's head 225 is detected, and completion of step 122 determines that the infant's head 225 is approximately positioned at the centre of the area 200. Step 124 then determines that region of interest 230 to comprise a rectangular bounding box that is approximately four times the area of the infant's head 225 and position so that the centre of the region of interest 230 substantially corresponds to the centre of the infant's head 225. That is, the region of interest 230 is determined to be a rectangular region that is slightly larger in area than the infant's head 225 (i.e. encompasses the infant's head) and is positioned centrally within the area 200 (thus aligning with the generally central location of the infant's head).

Here, it is noted that region of interest is depicted in Figure 2 as being a special type of rectangle, namely a square. The region of interest may, however, be determined to have a different shape in other embodiments. For example, the region of interest may have a regular polygonal shape (e.g. square, rhombus, equilateral triangle, etc.) or an irregular polygonal shape (e.g. scalene triangle, right triangle, isosceles triangle, rectangle, parallelogram, irregular pentagon, irregular hexagon, etc.). The shape of the region of interest may, for example, depend on the nature or type of the detected feature.

To provide further context for the determination of the exemplary region of region of interest 230 shown in Fig. 2, it is noted that, for analysis of breathing rate of the infant, it is typically preferable to analyze (and monitor) image data of an infant's chest region. Thus, the region of interest may be determined so that it includes the chest region and the region of interest then split into a plurality of blocks (i.e. segmented into a regular plurality of sub-squares) so to perform the pixel shift analysis. Having multiple blocks enables more robust analysis, because the direction of shift analysis in the chest region can vary due to the nature of belly/chest moving in different directions, e.g. upper blocks of chest moving up and down, but close to the belly area i.e. moving left and right. Thus, for embodiments, one may prefer to locating the chest area/region of the infant. However, considering all kind of user situations, training a feature detection for an infant's chest area/region may be difficult. For instance, an infant's chest area/region may be covered by clothing or a blanket. Hence, in exemplary embodiments, the feature detection model is described as being trained to identify an infant's head (as it may have a greater chance of remaining visible at all times).

Based on the infant head detection, the region of interest 230 in the example of Fig. 2 is a bounding box positioned around (i.e. overlapping and encompassing) the infant's head. This region of interest 230 is split into smaller blocks and pixel shift analysis may then be performed every frame on every block. All the individual smaller blocks can be analyzed and, based on periodicity of shift signal, a selection of blocks may be used and combined into one shift signal from which the breathing metrics are analyzed.

Restriction of processing/analysis to the region of interest 230 in this way may serve two main purposes: (I) It requires less computational power (e.g. 20% compared to full HD analysis); and (II) Due to the common use of wide angle cameras, a lot of surroundings are captured by the camera, containing a lot of contrast/spatial information (with a high risk of false shift signals). By restricting analysis to the region of interest (e.g. cropping data to the region of interest only), one may exclude non-relevant video data from breathing/sleep analysis.

In the above outlined method, the feature detection model is trained to detect a head of an infant, but the proposed invention is not limited to such. For instance, the feature detection model feature detection model may be trained to identify one or more other body parts of an infant, such: an eye; a nose; an ear; a mouth; a head; a shoulder; a torso; an arm; a leg; a knee; an ankle; a hand; and a foot. The region of interest may then be appropriately determined according to a desired/preferred monitoring region location relative to the position of such a detected feature.

Also, the first image data may not be a frame of a video taken from a video baby monitor but may comprise an image taken by a webcam or smartphone.

The feature detection model may employ one or more machine-learning algorithms. A machine-learning algorithm is any self-training algorithm that processes input data in order to produce or predict output data. Here, the input data comprises image data of a monitored area which may include an infant bed or crib and the output data comprises a prediction of one or more features of the infant's body detected in the image data.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives. Purely by way of example, one or more of the machine learning models may employ a Convolution Neural Network, because CNNs have been proven to be particularly successful at analyzing images, and being able to identify features/objects within images, with a much lower error rate than other types of neural network.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). Some embodiments of the present invention may employ CNN-based learning algorithms, because CNNs have proved to be particularly successful at analyzing images, and are able to identify features/objects within images with a much lower error rate than other types of neural network.

CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ± 1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

The training input data entries for the object detection model correspond to example images of an infant laying in a bed. The training output data entries correspond to the co-ordinates of the bounding boxes representing the spatial location of the feature(s) of the infant's body in the training image. In other words, the feature detection model may be trained using a using a deep learning algorithm configured to receive an array of training inputs and respective known outputs, wherein a training input comprises training image data of an infant laying or sleeping in a cot or bed and a respective known output. In this way, the feature detection model may be trained to output a prediction of the spatial location of one or more features of the infant's body parts (e.g. head and/or upper torso) when provided with an image of a lying infant.

The feature detection model may be a pre-trained model further trained on images of a laying or sleeping infant that have been manually annotated. For example, short videos may be taken of laying infants from various angles. Images may then be extracted from the videos in order to serve as training inputs with known infant positions. This allows the models to become especially proficient at predicting the position of one or more features of the infant's body, regardless of image capture (i.e., camera) position.

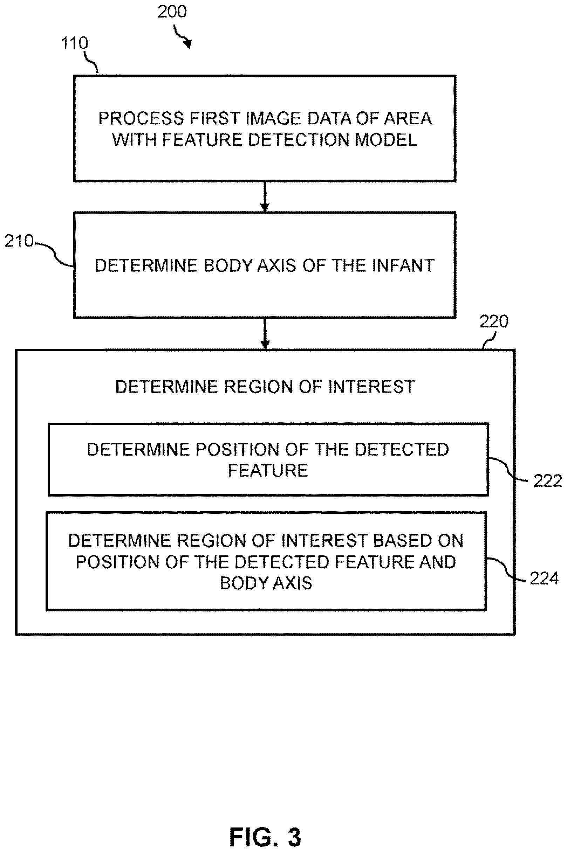

Referring now to Fig. 3 there is depicted a simplified flow diagram of a method 200 for determining a region of interest according to another embodiment. Here, the method 200 is similar to the method 100 of Fig. 1 but differs in that it is configured to determine the region of interest further based on a body axis of the infant. That is, the method of 200 seeks to define the region of interest in consideration of a direction of the body axis of the infant, wherein the body axis describes the longitudinal direction of the upper torso of the infant.

Like with the method 100 of Fig. 1, the method 200 of Fig. 2 begins with the step 110 of processing first image data of the area with a feature detection model to detect a feature of the infant's body. Again, the first image data, in this embodiment, is a frame of a video taken by a video baby monitor. Also, the feature detection model in this exemplary embodiment is an AI model trained to detect the presence of an infant's head in the image of the area.

Once the feature of the infant (i.e. the infant's head) has been detected, the method proceeds to a step 210. Step 210 comprises determining a body axis based on at least one of the detected feature and the first image data. Here, any suitable known methods for determining the body axis of the infant may be employed.

Purely by way of example, step 210 of this embodiment employs a previously proposed concept for determining a body axis of a laying infant as presented in International Patent Application Publication Number WO2024126318A1. One such exemplary concept presented in WO2024126318A1 includes a high-level process for predicting body posture of a laying infant from an image of laying infant (e.g. a frame of a captured video). The process includes steps where AI models predict if an infant is present in the captured image (e.g. whether the infant is in the crib/cot), detecting the relevant boxes and keypoints, and finally predicting the body posture of the laying infant. Using an object detection AI model, the location of infant head and upper body bounding boxes is predicted. The video frame is then provided to a keypoint detection AI model which determines the location of keypoints along with the location of head and upper body centre of masses (COMs). Based on the COMs, the body axis AB is determined (e.g. defined by a line passing through the head and upper body COMs), wherein an orientation of the body axis AB relative to the viewing direction (i.e. viewpoint) of the image may be inferred/determined from the head to upper body direction. For instance, the orientation of body axis may be defined with respect to a direction of travel along axis from the head to the upper body. In this way, the body axis may represent a longitudinal direction of the upper torso of the infant and also the orientation of the upper torso with respect to the viewpoint of the image

The method then proceeds to step 220, which comprises determining, based on the position of the detected feature within the area and the body axis, a region of interest within the area.

In this exemplary embodiment, step 220 comprises the sub-steps 222 and 224. Step 222 comprises determining the position of the detected feature within the area, and step 224 comprises determining the region of interest based on the position of the detected feature (as determined in step 222) and the body axis (as determined in step 210).

By way of example, step 224 of determining a region of interest within the area comprises determining a measure of an orientation of the region of interest based on at least one of the detected feature and the determined body axis. Here, the measure of the orientation of the region of interest comprises at least one of: portrait with respect to a horizontal axis of the area (e.g. an x-axis that is parallel with a long edge of the image data and/or area); landscape with respect to the horizontal axis of the area; and an angle between an axis of the region of interest (e.g. a central axis of symmetry of the region of interest, or a longitudinal axis of the region of interest) and the horizontal axis of the area.

That is, in step 224, an orientation angle of the region of interest is determined based on the determined body axis of the infant.

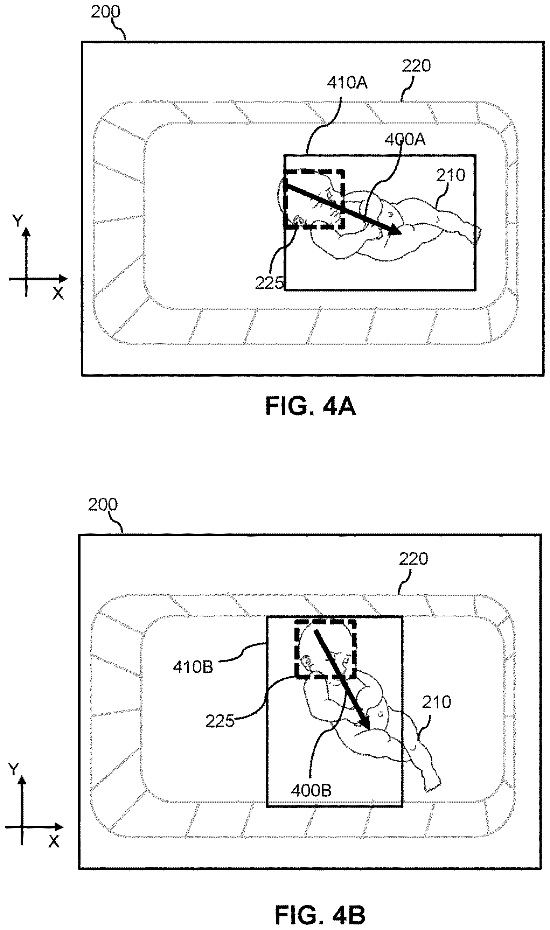

For example, as illustrated in Figs. 4A-4D, the area 200 in which an infant 210 is to be monitored corresponds to a field of view of the video baby monitor (and thus the field of view of first image data). The horizontal X and vertical Y axes are illustrated with respectively labelled arrows, wherein the horizontal axis X is parallel with the long edge of the area 200, and wherein the vertical axis Y is parallel with the short edge of the area 200. In the field of view (and thus within the field of view of first image data) is a cot 220 within which the infant 210 is laying.

In the example shown by Fig. 4A, by processing the first image data according to step 110, the infant's head 225 is detected, and completion of step 122 determines that the infant's head 225 is approximately positioned at the centre of the area 200. Also, according to step 210, the body axis 400A of the infant head 225 is determined to be in a (shallow) downwards right direction (as indicated by the arrow labelled "400A"), i.e. the angle formed between the body axis and the horizontal axis X is less than 45°. Step 224 then determines that region of interest 410A to comprise a rectangular bounding box that is approximately four times the area of the infant's head 225 and positioned so that the centre of the region of interest 410A is offset from the centre of the infant's head 225 in a direction that is parallel with the body axis 400A (but with the infant's head still contained within the region of interest 410A). That is, the region of interest 410A is determined to be a rectangular region that is larger in area than the infant's head 225 (i.e. encompasses the infant's head) and with its central point translated parallel to the body axis 400A. In this way, the torso of the infant is positioned substantially centrally within the region of interest 410A.

In the example shown by Fig. 4B, by processing the first image data according to step 110, the infant's head 225 is detected, and completion of step 122 determines that the infant's head 225 is approximately positioned centrally along the horizontal X axis of the area 200, and vertically above the centre of the area 200 in the vertical Y axis. That is, the infant's head may be said to be in an upper central position of the area 200. Also, according to step 210, the body axis 400B of the infant head 225 is determined to be in a (steep) downwards right direction (as indicated by the arrow labelled "400B") i.e. the angle formed between the body axis and the vertical axis Y is less than 45°. Step 224 then determines that region of interest 410B to comprise a rectangular bounding box that is approximately four times the area of the infant's head 225 and positioned so that the centre of the region of interest 410B is offset from the centre of the infant's head 225 in a direction that is parallel with the body axis 400B (but with the infant's head still contained within the region of interest 410B). That is, the region of interest 410B is determined to be a square region that is larger in area than the infant's head 225 (i.e. encompasses the infant's head) and with its central point translated parallel to the body axis 400B. In this way, the torso of the infant is again positioned substantially centrally within the region of interest 410B.

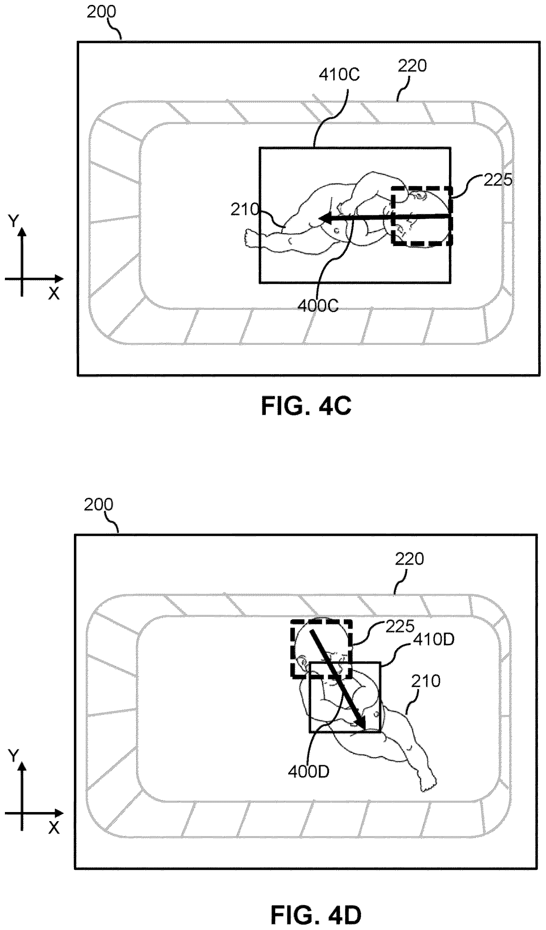

In the example shown by Fig. 4C, by processing the first image data according to step 110, the infant's head 225 is detected, and completion of step 122 determines that the infant's head 225 is approximately positioned at the centrally along the vertical Y axis of the area 200, and to the right of centre of the area 200 in the horizontal X axis. That is, the infant's head may be said to be in a middle right position of the area 200. Also, according to step 210, the body axis 400C of the infant head 225 is determined to be in a horizontal left direction (as indicated by the arrow labelled "400C") i.e. the angle formed between the body axis and the horizontal axis X is 0° and the angle formed between the body axis and the vertical axis Y is 90°. Step 224 then determines that region of interest 410C to comprise a rectangular bounding box that is approximately four times the area of the infant's head 225 and positioned so that the centre of the region of interest 410C is horizontally offset to the left from the centre of the infant's head 225 (i.e. in a direction that is parallel with the body axis 400C), with the infant's head still contained within the region of interest 410C). That is, the region of interest 410C is determined to be a rectangular region that is larger in area than the infant's head 225 (i.e. encompasses the infant's head) and with its central point translated parallel to the body axis 400C. In this way, the torso of the infant is once again positioned substantially centrally within the region of interest 410C.

In the example shown by Fig. 4D, by processing the first image data according to step 110, the infant's head 225 is detected, and completion of step 122 determines that the infant's head 225 is approximately positioned centrally along the horizontal X axis of the area 200, and vertically above the centre of the area 200 in the vertical Y axis. That is, the infant's head may be said to be in an upper central position of the area 200. Also, according to step 210, the body axis 400D of the infant head 225 is determined to be in a (steep) downwards right direction (as indicated by the arrow labelled "400D") i.e. the angle formed between the body axis and the vertical axis Y is less than 45°. Step 224 then determines that region of interest 410D to comprise a rectangular bounding box that is approximately one and a half (1.5) times the area of the infant's head 225 and positioned so that the centre of the region of interest 410D is offset from the centre of the infant's head 225 in a direction that is parallel with the body axis 400D (but with the infant's head only partially overlapping the region of interest 410D). That is, the region of interest 410D is determined to be a square region that is only slightly larger in area than the infant's head 225 and with its central point translated parallel to the body axis 400D. In this way, the torso of the infant is positioned substantially centrally within the region of interest 410D (and the region of interest 410D corresponds roughly in size with the torso of the infant).

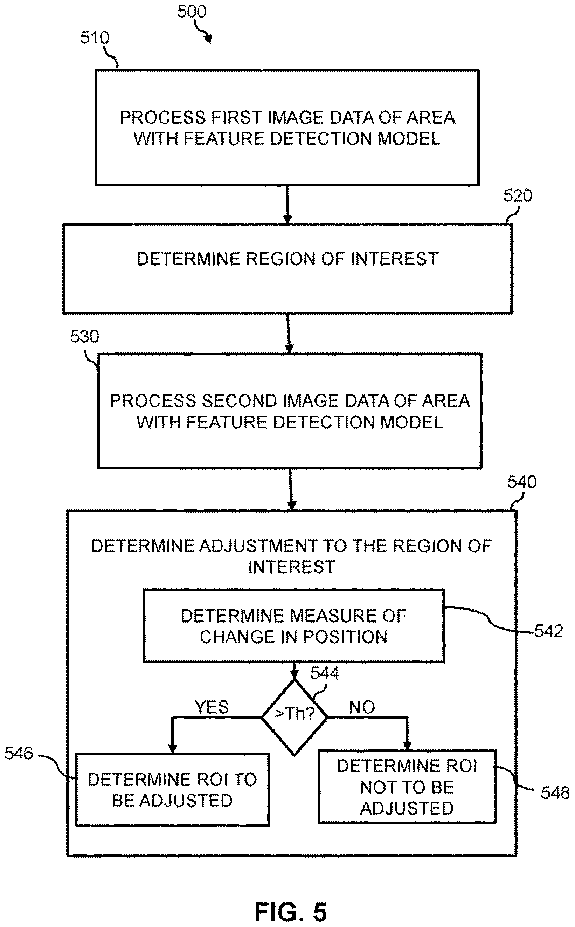

Referring now to Fig. 5 there is depicted a simplified flow diagram of a method 500 for determining a region of interest according to yet another embodiment. Here, the method is further configured to modify or adapted the region of interest according to a temporal change in the feature. For instance, the method of this embodiment may change/update the region of interest according to changes in the position and/or orientation of the feature of the infant over time.

The method begins with a step 510, comprising processing first image data of the area with a feature detection model to detect a feature of the infant's body. The first image data, in this embodiment, is a frame of a video taken by a video baby monitor and thus associated with a first image capture time t1, i.e. the time of capture of the video frame. Also, the feature detection model in this exemplary embodiment is an AI model trained to detect the presence of an infant's head in the image of the area. Any well-known suitable object detection model may be used, for example YOLO, R-CNN, Mask R-CNN, MobileNet, or Squeeze Det.

Once the feature of the infant (i.e. the infant's head) has been detected, the method proceeds to a step 520. Step 520 comprises determining, based on the position of the detected feature within the area, a region of interest within the area. This region of interest is a region to be used for monitoring the infant.

The method then proceeds to step 530. Step 530 comprises processing second image data of the area with the feature detection model to detect the feature of the infant's body in the second image data. Here, the second image data is a subsequent (i.e. later captured) frame of the video taken by the video baby monitor, and thus the second image data is associated with a second image capture time t2 that is after the first image capture time t1. Put another way, the second image data is captured a time t2 that occurs after the capture time t1 of the first image data. Also, it will be appreciated that, because the first image data and the second image data are different frames of the same video, the first image data and the second image data of this embodiment are from a single image sensor.

Once the feature of the infant (i.e. the infant's head) has been detected in the second image data, the method proceeds to a step 540. Step 540 comprises determining, based on the position of the feature detected in the second image data, an adjustment to the region of interest.

In this exemplary embodiment, step 540 comprises the sub-steps 542, 544, 546 and 548.

Step 542 comprises determining a measure of change in position of the feature detected in the first and second image data. That is, the position of the infant's head detected in the second image data is compared with the position of the infant's head in the first image data, thus establishing a change of position of the infant's head between the two capture times t1 and t2. The change of position may, for example, may be described by a vector establishing a distance and direction moved by the infant's head between the two capture times t1 and t2. Alternatively, the measure of the change of position may be described using an absolute value (e.g. magnitude of distance moved) or a relative value (e.g. percentage of the area width or length).

Next, in step 544, the measure of change in position is compared with a threshold value Th to determine if the region of interest is to be adjusted. Specifically, if the measure of the change in position is greater than a predetermined threshold value Th, the method proceeds to step 546 in which it is determined that the region of interest is to be adjusted and the adjustment is then determined. Responsive the completion of step 546, the method may generate an output signal comprising information describing the determined adjustment. The output signal may then be used (e.g. by a control unit/system) to adjust the region of interest according to information contained therein (i.e. according to the adjustment determined by step 546).

Conversely, if the measure of the change in position is less than the predetermined threshold value Th, the method proceeds to step 548 in which it is determined that the region of interest is not to be adjusted.

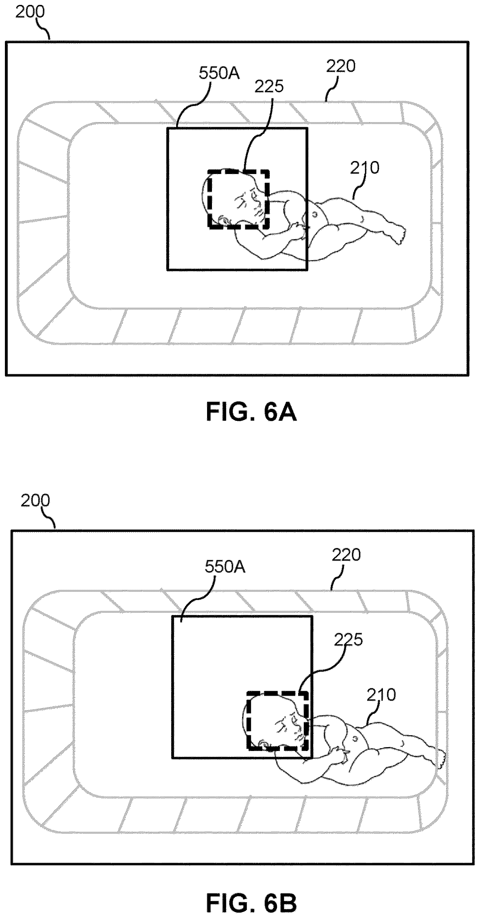

An exemplary implementation of the method of Fig. 5 will now be described with reference to Figs. 6A-6C.

Fig. 6A depicts an area 200 at a first image data capture time t1. An infant 210 to be monitored is laying in a cot 220 at the first image data capture time t1. As illustrated in Fig. 6A, the area 200 in which the infant 210 is to be monitored corresponds to a field of view of a video baby monitor (and thus the field of view of first image data). Within the field of view (and thus within the field of view of first image data) is the cot 220 within which the infant 210 is laying. By processing the first image data captured at the first image data capture time t1 according to step 510, the infant's head 225 is detected. Step 520 then determines that region of interest 550A to comprise a square or rectangular bounding box that is approximately four times the area of the infant's head 225 and positioned so that the centre of the region of interest 550A substantially corresponds to the centre of the infant's head 225. That is, the region of interest 550A is determined to be a square region that is slightly larger in area than the infant's head 225 (i.e. encompasses the infant's head) and is positioned centrally within the area 200 (thus aligning with the generally central location of the infant's head).

Fig. 6B depicts the area 200 at a second image data capture time t2 that is after the first image data capture time t2 (e.g. t2 is approximately 30 seconds later than t1). The infant 210 to be monitored is still laying in the cot 220 at the second image data capture time t2. Again, as illustrated in Fig. 6B, the area 200 in which the infant 210 is to be monitored corresponds to the field of view of a video baby monitor (and thus the field of view of second image data). That is, the field of view of a video baby monitor has not changed between the first t1 and second t2 image data capture times, i.e. the image sensor has remained stationary over time.

By processing the second image data captured at the second image data capture time t2 according to step 530, the infant's head 225 is detected and it is determined that the infant's head 225 is approximately positioned at lower right corner of the region of interest 550A. That is, it is determined that the infant's head has moved down and to the right within the region of interest. The method proceeds to a step 540 of determining, based on the position of the feature detected in the second image data, an adjustment to the region of interest 550A.

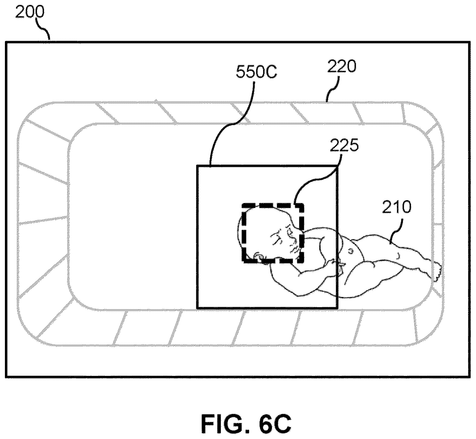

Specifically, step 540 determined that the region of interest 550A is to be adjusted by translating it down and right in a manner substantially matching the change in position of the infant's head between the first t1 and second t2 image data capture times. By matching the adjustment of the region of interest 550A with the determined change in position of the infant's head, a new, adjusted region of interest 550C is positioned so that the centre of the new, adjusted region of interest 550C substantially corresponds to the centre of the infant's head 225 in the second image data. This is illustrated in Fig. 6C which illustrates the area 200 after adjustment of the adjusted region of interest 550C is determined by completion of step 540 and subsequently implemented by an exemplary embodiment. That is, the exemplary implementation includes a further step that affects adjustment of the region of interest so that the adjusted region of interest 550C is positioned so that its centre point is aligned with the central location of the infant's head 225.

By way of further example of the proposed adjustment concept, another embodiment may be configured to only adjust the region of interest if the infant moves greater than 25% of the width/height distance from the original centre of the region of interest. This means that, for a 3200x3200 region of interest, if the feature of the infant moves more than 80 pixels away from its originally detected x or y position, the region of interest is repositioned. This percentage-based threshold value may, of course, employ other values and/or adapted according to needs. However, experimentation has identified that the 25% value achieves a good compromise between not moving the region of interest too frequently (causing a lot of reinitiation of breathing analysis) and ensuring sufficient breathing information in the region of interest (e.g. maintaining the infant's chest area within the region of interest).

Other embodiments may, however, user other references to determine if the region of interest is to be adjusted. For example, a measure of a new position of the feature may be defined relative to any one of various references, such as a previously detected position of the feature, an edge/boundary of a previously defined region of interest, or the centre point of a previously defined region of interest. That is, a newly detected position of the feature may be checked against refence value or position to determine if the region of interest requires updating.

### Example (i):

For first image data capture time t1 - Detect the infant's head and define a region of interest around it.

For second image data capture time t2 - Detect the infant's head again and check if the region of interest needs updating. If the new position of the infant's head is less than 25% away from a edge of the previously defined region of interest, update the region of interest.

### Example (ii):

For first image data capture time t1 - Detect the infant's head and define a region of interest around it.

For second image data capture time t2 - No infant head can be detected. Region of interest maintained as previously defined (i.e. no change to the region of interest).

For third image data capture time t3 (t2 being after t3) - If the new infant head position is detected at a distance greater than 25% from center of the previously defined region of interest, update the region of interest location.

In the above outlined method, the feature detection model is trained to detect a head of an infant, but embodiments need not be limited to such. For instance, the feature detection model feature detection model may be trained to identify one or more other body parts of an infant, such: an eye; a nose; an ear; a mouth; a head; a shoulder; a torso; an arm; a leg; a knee; an ankle; a hand; and a foot or other relevant features such as a mattress area. The region of interest may then be appropriately determined according to a desired/preferred monitoring region location relative to the position of such a detected feature.

Also, any suitable image data of the target area to which feature detection models can be applied may be used as the image data. This includes any image or images taken of the target area from any capable device. Thus, the first and second image data need not be the same and may indeed be in different forms. Similarly, the first and second image data may be taken by the same or different image sensors. Using the same image sensor to acquire both sets of data has the advantage that it reduces the number of sensors required to be included in a suitable monitoring device and further ensures that this method can be integrated into existing infant monitoring systems.

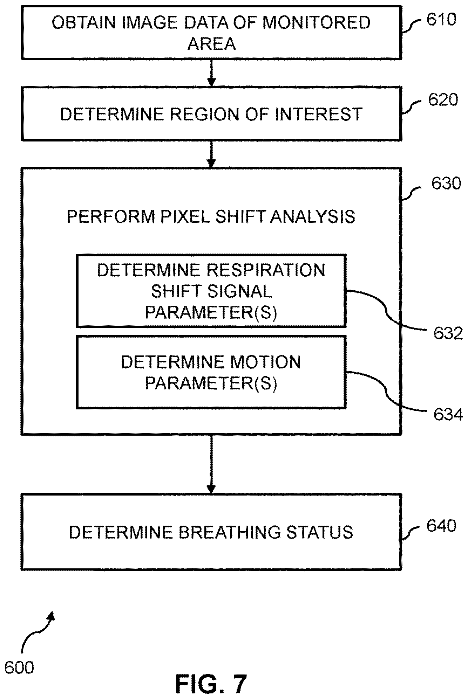

Referring now to Fig. 7 there is depicted a simplified flow diagram of a method 600 for determining the breathing status of an infant in a monitored area, wherein the method employs a method for determining a region of interest according to a proposed embodiment. This example depicts in more detail how an embodiment may be leveraged when determining the breathing status of an infant in a monitored area.

The method starts with a step 610 which comprises obtaining image data of the area being monitored. This may be taken by an infant monitoring device equipped with an image sensor and positioned above the bed or crib of an infant such as to obtain a birds-eye view of the sleeping area of the infant. The image data is then provided to a processing arrangement configured to process the image data with a feature detection model to detect a feature of the infant's body.

The method then proceeds to a step 620 which comprises determining, based on the position of the detected feature within the area, a region of interest within the area, wherein the region of interest is a region to be used for monitoring the infant. In this exemplary embodiment, the total area depicted in the image data is cropped to the region of interest so as to obtain cropped image data of a smaller area corresponding to the region of interest. For example, the region of interest may be configured so that it equates to a 720p crop taken from a full HD image.

The step of cropping the image data to the determined region of interest described herein reduces the computational power required to process video data and allows the irrelevant or lower priority image data to be filtered out which may otherwise result in false motion detection signals.

Once the region of interest has been defined the method moves to a step 630 comprising performing pixel shift analysis on video data cropped to the region of interest. This involves analysing at least two frames of the cropped video data, segmenting the cropped area into smaller blocks of, for example, 32 by 32 pixels, and identifying any texture shifts between corresponding pixel blocks in the two frames. The pixel shift analysis may be carried out on every frame of the video data, wherein the frame rate of the video data can range between 8 and 60 fps. The pixel shift analysis results in the detection of both periodic and non-periodic motion in the target area. The pixel shift analysis step 630 further comprises the sub-steps 632 and 634.

Step 632 comprises determining at least one respiration shift signal parameter, wherein the at least one respiration shift signal parameter describes at least one characteristic of any detected periodic motion that is likely to relate to breathing of an infant in the target area. In this exemplary embodiment, the at least one respiration shift signal parameter comprises: a shift signal amplitude describing the average amplitude of detected texture shifts, a spectral flatness parameter describing the level of noise of the detected shifts, and a breathing confidence metric describing the regularity of the periodicity of the detected periodic motion.

Step 634 involves determining at least one motion parameter describing any non-periodic motions detected in the cropped video data which may relate to an infant moving or rolling-over in the target area. In this exemplary embodiment this involves averaging the texture shifts detected across all the pixel blocks analysed. If the average texture shift exceeds a given threshold it is determined that a large motion has occurred. If any large motions have occurred in the last thirty (30) seconds there is a high likelihood that an infant is in the target area at the present instance.

Finally, in step 640, the breathing status of the infant is determined based on the at least one respiration shift signal parameter and the at least one motion parameter.

It is noted that although embodiments described above have defined square-shaped regions of interest (and also illustrated segmenting a region of interest into smaller blocks of, for example, 32 by 32 pixels), a region of interest determined according to a proposed embodiment need not be limited to being square in shape. That is, in other embodiments, a determined region of interest may be of different shaped and/or sizes, according to predetermined requirement for example.

By way of example, an area of the region of interest; a dimension of the region of interest; and/or the proportion of the area covered by the region of interest may be determined may be determined by embodiments, and such determination may be based on various aspects such as the detected feature of the infant and/or a body axis of the infant. In this way, a region of interest may be defined so as to closely match an outline of the infant's body, for example.

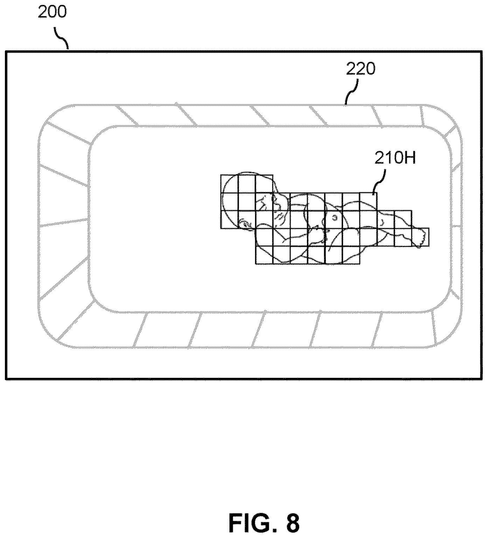

Indeed, as illustrated in Fig. 8, a region of interest 210H determined according to an embodiment may comprise a plurality of square segments that are arranged in a pattern that closes matched the shape of the infant's body. To determine at least one of the shape, size and orientation of such a region of interest 210H, proposed methods may include a step of determining a boundary of the region of interest, which may, in turn, comprise processing the image data with at least one of: an object detection model; an edge detection model; and a contrast enhancement model, e.g. in order to identify edges of the infant's body in the image data.

Proposed embodiments may therefore employ a combination of a plurality of different detection models to determine the region of interest. By employing different detection models in combination, different image data analysis approaches may be leveraged to increase and/or improve the information used for determining the region of interest, thus improving reliability and/or accuracy.

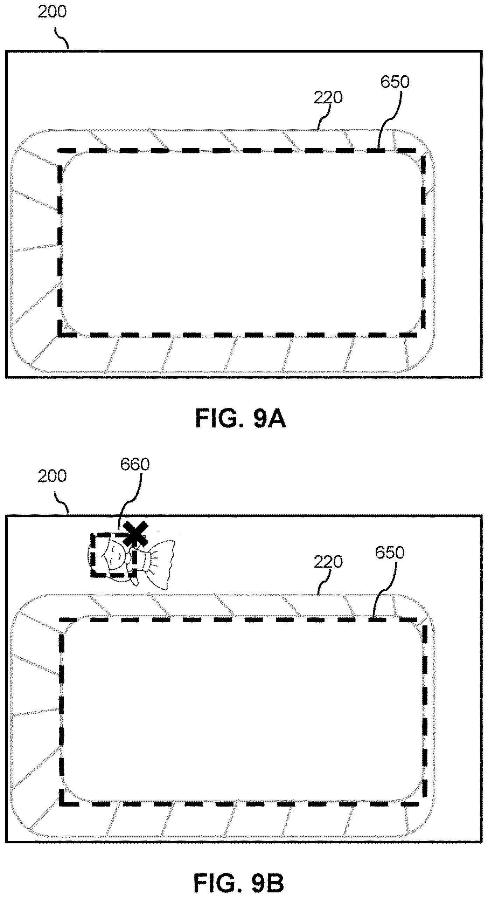

Purely by way of further example of how determining the region of interest may employ various information, Figs. 9A-9D illustrate another exemplary implementation concept wherein it is checked if a region of interest is within a predetermined region of the area being monitored (the specific region in this example corresponding to the area of a mattress within a cot). More generally, a validity of a candidate region of interest is determined by checking whether its location within the monitored area meets a predetermined requirement.

Fig. 9A depicts an area to be monitored at a first image data capture time t1. As illustrated in Fig. 9A, the area 200 in which an infant is to be monitored corresponds to a field of view of a video baby monitor (and thus the field of view of first image data). Within the field of view (and thus within the field of view of first image data) is a cot 220 containing a mattress. The outer edge of the mattress is indicated in Fig. 9A by a mattress boundary box 650. The mattress boundary box 650 may be determined by employing one or more detection models or may be defined by a user input (e.g. marked out by a user via a graphical user interface).

Fig. 9B depicts the area 200 at a second image data capture time t2 that is after the first image data capture time t2 (e.g. t2 is approximately 1 minute later than t1). Again, the area 200 in which an infant is to be monitored corresponds to the field of view of a video baby monitor (and thus the field of view of second image data). That is, the field of view of a video baby monitor has not changed between the first t1 and second t2 image data capture times, i.e. the image sensor has remained stationary over time.

At the second image data capture time t2, there is no infant laying in the cot, but there is toy doll positioned outside of the cot 220 (and within the area 200).

By processing the second image data captured at the second image data capture time t2, the head of the toy doll is detected and a candidate region of interest 660 is determined. Specifically, it is determined that the candidate region of interest 660 comprises a rectangular bounding box that is approximately one and half times the area of the toy doll's head and is positioned so that the centre of the candidate region of interest 660 substantially corresponds to the centre of the toy doll's head. That is, the candidate region of interest 660 is determined to be a rectangular region that is slightly larger in area than the toy doll's head 225 (i.e. encompasses the toy doll's head) and is positioned within the upper left region of the area 200 outside of the mattress boundary box 650. Consequently, a check to determine if the candidate region of interest 660 is within a predetermined region of the area (the predetermined region being the mattress boundary box 650) results in a negative finding. It is determined that the candidate region of interest 660 does not meet the requirement that it must be within the mattress boundary box 650. The candidate region of interest 660 is then rejected/discarded (as indicated in Fig. 9B by the large cross depicted adjacent the candidate region of interest 660).

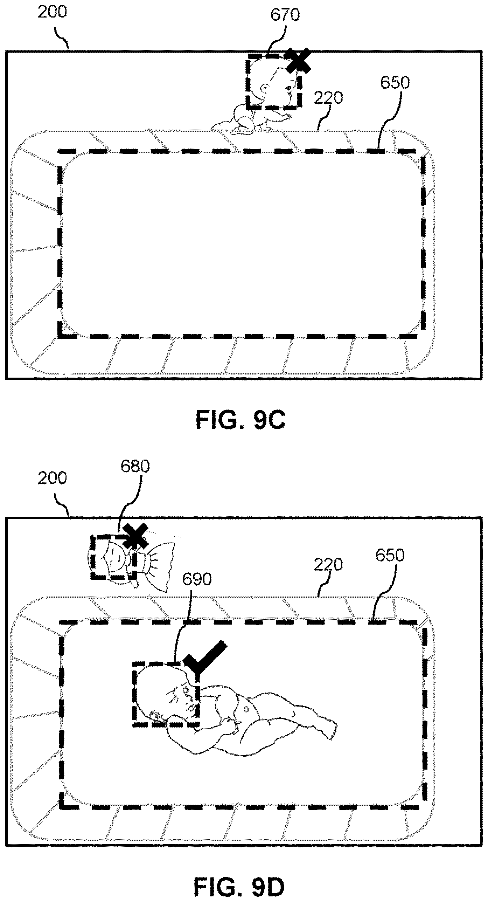

Fig. 9C depicts the area 200 at a third, different image data capture time t3 (i.e. t3≠t1 and t3^ t2). Once again, the field of view of a video baby monitor at the third image data capture time t3 matches that of the first t1 and second t2 image data capture times, i.e. the image sensor has remained stationary. At the third image data capture time t3, there is no infant laying in the cot, but there is a crawling infant positioned outside of the cot 220 (and within the area 200).

By processing the third image data captured at the third image data capture time t3, the head of the crawling infant is detected and a candidate region of interest 670 is determined. Specifically, it is determined that the candidate region of interest 670 comprises a rectangular bounding box that has approximately the same area as the crawling infant's head and is positioned so that the centre of the candidate region of interest 670 substantially corresponds to the centre of the crawling infant's head. That is, the candidate region of interest 670 is determined to be positioned within the upper middle region of the area 200 outside of the mattress boundary box 650. Consequently, a check to determine if the candidate region of interest 670 is within the predetermined region of the area (the predetermined region being the mattress boundary box 650) results in a negative finding. It is determined that the candidate region of interest 670 does not meet the requirement that it must be within the mattress boundary box 650. The candidate region of interest 670 is then rejected/discarded (as indicated in Fig. 9C by the large cross depicted adjacent the candidate region of interest 660).

Fig. 9D depicts the area 200 at a fourth, different image data capture time t4 (i.e. t4≠t1, t4≠t2 and t4^ t3). Yet again, the field of view of a video baby monitor at the fourth image data capture time t4 matches that of the first t1, second t2 and third t3 image data capture times, i.e. the image sensor has remained stationary. At the fourth image data capture time t4, there is an infant laying in the cot, and there is toy doll positioned outside of the cot 220 (and within the area 200).

By processing the fourth image data captured at the fourth image data capture time t4, the head of the toy doll is detected and first 680 and second 690 candidate regions of interest are determined.

Specifically, it is determined that the first candidate region of interest 680 comprises a rectangular bounding box that is approximately one and half times the area of the toy doll's head and is positioned so that the centre of the first candidate region of interest 680 substantially corresponds to the centre of the toy doll's head. That is, the first candidate region of interest 680 is determined to be a rectangular region that is slightly larger in area than the toy doll's head 225 (i.e. encompasses the toy doll's head) and is positioned within the upper left region of the area 200 outside of the mattress boundary box 650. Consequently, a check to determine if the first candidate region of interest 680 is within the predetermined region of the area (the predetermined region being the mattress boundary box 650) results in a negative finding. It is determined that the first candidate region of interest 680 does not meet the requirement that it must be within the mattress boundary box 650. The first candidate region of interest 680 is then rejected/discarded (as indicated in Fig. 9D by the large cross depicted adjacent the first candidate region of interest 660).

Further, it is determined that the second candidate region of interest 690 comprises a rectangular bounding box that has approximately the same area as the laying infant's head and is positioned so that the centre of the second candidate region of interest 690 substantially corresponds to the centre of the laying infant's head. That is, the second candidate region of interest 690 is determined to be positioned slightly left of the centre of the area 200 (and inside of the mattress boundary box 650). Consequently, a check to determine if the second candidate region of interest 690 is within the predetermined region of the area (the predetermined region being the mattress boundary box 650) results in a positive finding. It is determined that the second candidate region of interest 690 does meet the requirement that it must be within the mattress boundary box 650. The second candidate region of interest 690 is then accepted a being valid (as indicated in Fig. 9D by the large checkmark depicted adjacent the second candidate region of interest 690).

From the examples described above with reference to Figs. 9A-9D, it will be understood that embodiments may employ one or more concepts of checking the validity of a candidate (i.e. potential) region of interest, and such concepts may employ one or more conditions or requirements that a required to be met. This may help to reduce false-positives and improve accuracy.

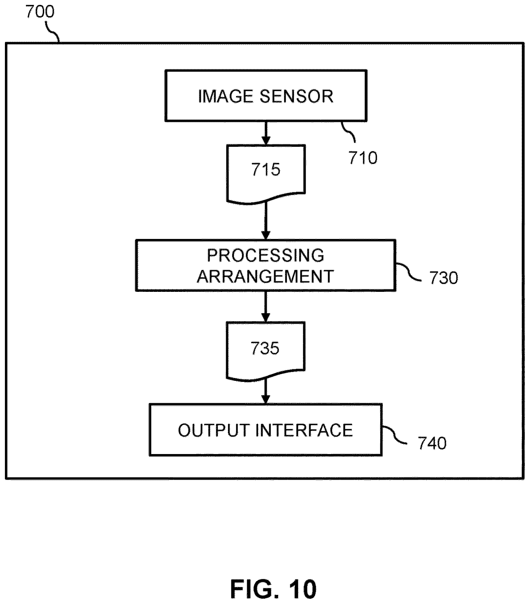

Referring now to Fig. 10, there is depicted a simplified block diagram of an infant monitoring system 700 for use in monitoring an infant within an area according to a proposed embodiment. The infant monitoring system comprises an image sensor 710, a processing arrangement 730, and an output interface 740. The processing arrangement unit 730 may also be referred to as an image processor.

The system 700 is configured to determine a region of interest for use in monitoring an infant within the area being monitored by the system. It achieves this by processing in the processing arrangement unit 730 image data from the image sensor 710. This may be carried out using the method 100 depicted in Fig. 1 for example.

In more detail, the image sensor 710 produces image data 715 of the area to be monitored. The image data from the image sensor is sent to the processing arrangement unit 730.

The processing arrangement unit 730 is configured to run computer code comprising code means for implementing the method 100. Thus. On receiving the image data 715 the processing arrangement unit 730 processes the image data of the area with a feature detection model to detect a feature of the infant's body. The processing arrangement unit 730 then determines, based on the position of the detected feature within the area, a region of interest within the area, wherein the region of interest is a region to be used for monitoring the infant.

The processing arrangement unit 730 is configured to output an indication 735 of the region of interest 735 to the output interface 740 which then may output this indication to the user and/or an infant monitoring process (e.g. as an electronic output signal and/or a visible/audible output).

The method 100 may be performed on image data taken at regular time intervals to determine adjustments or medications to the region of interest. These determinations may then be used to automatically and dynamically adjust the region of interest (e.g. so as to cater for movement of the infant within the area over time).

Although in the system 700, the image sensor 710, the processing arrangement unit 730, and the output interface 340 are present in the same device, it will be understood that these components may be distributed among various devices. For example, there may be provided an infant monitor comprising only an image sensor. The image data taken by the image sensor of the image monitor may then be relayed to a personal smart device of the parent/carer. The processing and interface capacities of the personal smart device may then fulfil the functionalities of the processing arrangement unit 730 and the output interface 740.

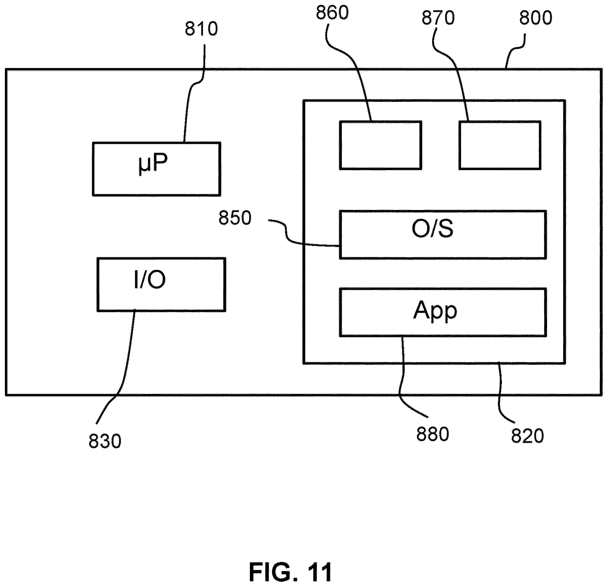

Fig. 11 illustrates an example of a computer 800 within which one or more parts of an embodiment may be employed. Various operations discussed above may utilize the capabilities of the computer 800. In this regard, it is to be understood that system functional blocks can run on a single computer or may be distributed over several computers and locations (e.g. connected via internet).

The computer 800 includes, but is not limited to, PCs, workstations, laptops, PDAs, palm devices, servers, storages, and the like. Generally, in terms of hardware architecture, the computer 800 may include one or more processors 810, memory 820 and one or more I/O devices 830 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 810 is a hardware device for executing software that can be stored in the memory 820. The processor 810 can be virtually any custom made or commercially available processor, a central processing unit (CPU), a digital signal processor (DSP), or an auxiliary processor among several processors associated with the computer 800, and the processor 810 may be a semiconductor-based microprocessor (in the form of a microchip) or a microprocessor.

The memory 820 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 820 may incorporate electronic, magnetic, optical, and/or other types of storage media. Note that the memory 820 can have a distributed architecture, where various components are situated remote from one another, but can be accessed by the processor 810.

The software in the memory 820 may include one or more separate programs, each of which comprises an ordered listing of executable instructions for implementing logical functions. The software in the memory 820 includes a suitable operating system (O/S) 850, compiler 860, source code 870, and one or more applications 880 in accordance with exemplary embodiments. As illustrated, the application 880 comprises numerous functional components for implementing the features and operations of the exemplary embodiments. The application 880 of the computer 800 may represent various applications, computational units, logic, functional units, processes, operations, virtual entities, and/or modules in accordance with exemplary embodiments, but the application 880 is not meant to be a limitation.

The operating system 850 controls the execution of other computer programs, and provides scheduling, input-output control, file and data management, memory management, and communication control and related services. It is contemplated by the inventors that the application 880 for implementing exemplary embodiments may be applicable on all commercially available operating systems.

Application 880 may be a source program, executable program (object code), script, or any other entity comprising a set of instructions to be performed. When a source program, then the program is usually translated via a compiler (such as the compiler 860), assembler, interpreter, or the like, which may or may not be included within the memory 820, so as to operate properly in connection with the O/S 850. Furthermore, the application 880 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 830 may include input devices such as, for example but not limited to, a mouse, keyboard, scanner, microphone, camera, etc. Furthermore, the I/O devices 430 may also include output devices, for example but not limited to a printer, display, etc. Finally, the I/O devices 830 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 830 also include components for communicating over various networks, such as the Internet or intranet.

If the computer 800 is a PC, workstation, intelligent device or the like, the software in the memory 820 may further include a basic input output system (BIOS) (omitted for simplicity). The BIOS is a set of essential software routines that initialize and test hardware at start-up, start the O/S 850, and support the transfer of data among the hardware devices. The BIOS is stored in some type of read-only-memory, such as ROM, PROM, EPROM, EEPROM or the like, so that the BIOS can be executed when the computer 800 is activated.

When the computer 800 is in operation, the processor 810 is configured to execute software stored within the memory 820, to communicate data to and from the memory 820, and to generally control operations of the computer 800 pursuant to the software. The application 880 and the O/S 850 are read, in whole or in part, by the processor 810, perhaps buffered within the processor 810, and then executed.

When the application 880 is implemented in software it should be noted that the application 880 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 880 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods and system shown in the Figures may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. Reference to `at least one of A and B' should be taken to cover each of: A; B; A and B. Similarly, reference to `A and/or B' should be taken to cover each of: A; B; A and B. In this way `at least one of and 'and/or' may be construed to be the same. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow diagrams, and combinations of blocks in the block diagrams and/or flow diagrams, can be implemented by special purpose hardware-based systems that perform the specified functions or acts or carry out combinations of special purpose hardware and computer instructions the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow diagrams, and combinations of blocks in the block diagrams and/or flow diagrams, can be implemented by special purpose hardware-based systems that perform the specified functions or acts or carry out combinations of special purpose hardware and computer instructions.

## Claims

1. A method for determining a region of interest for use in monitoring an infant within an area, the method comprising:
processing first image data of the area with a feature detection model to detect a feature of the infant's body; and
determining, based on the position of the detected feature within the area, a region of interest within the area, wherein the region of interest is a region to be used for monitoring the infant.

2. The method of claim 1, further comprising:
determining a body axis based on at least one of the detected feature and the first image data, wherein the body axis describes the longitudinal direction of the upper torso of the infant,
and wherein the step of determining a region of interest within the area is further based on the determined body axis.

3. The method of claim 2, wherein determining a region of interest within the area comprises determining a measure of at least one of a position, an orientation, size and shape of the region of interest based on at least one of the detected feature and the determined body axis.

4. The method of claim 3, wherein the measure of the orientation of the region of interest comprises at least one of: portrait with respect to a horizontal axis of the area; landscape with respect to a horizontal axis of the area; and an angle between an axis of the region of interest and the horizontal axis of the area.

5. The method of claim 3 or 4, wherein the measure of the size of the region of interest comprises at least one of: an area of the region of interest; a dimension of the region of interest; and the proportion of the area covered by the region of interest.

6. The method of any preceding claim, wherein the determining a region of interest comprises determining at least one of: a boundary; a size; and a validity of the region of interest,
and optionally wherein determining a at least one of: a boundary; a size; and a validity of the region of interest comprises processing the first image data with at least one of: an object detection model; an edge detection model; and a contrast enhancement model.

7. The method of any of claims 1 to 6, wherein the feature model is trained to detect at least one body part of the infant, and optionally wherein the at least one body part of the infant comprises at least one of: an eye; a nose; an ear; a mouth; a head; a shoulder; a torso; an arm; a leg; a knee; an ankle; a hand; and a foot.

8. The method of any of claims 1 to 7, further comprising:
processing second image data of the area with the feature detection model to detect the feature of the infant's body in the second image data, wherein first image data is associated with a first image capture time, and wherein the second image data is associated with a second image capture time subsequent to the first image capture time; and
determining, based on the position of the feature detected in the second image data, an adjustment to the region of interest,
and optionally wherein the first image data and the second image data are from a single image sensor.

9. The method of claim 8, wherein determining an adjustment to the region of interest comprises:
determining a measure of position of the feature detected in the second image data relative to a predetermined reference;
determining the region of interest is to be adjusted if the measure of position meets a predetermined condition; and
determining the region of interest is not to be adjusted if the measure of the position does not meet the predetermined condition,
and optionally wherein:
the predetermined reference is based on at least one of: the position of the feature detected in the first image data; the centre of the region of interest; and an edge of the region of interest; and
the predetermined condition requires that the measure of position exceeds a predetermined threshold value.

10. The method of any preceding claim, further comprising determining the region of interest is to be rejected if it does not meet a predetermined requirement, and optionally wherein the predetermined requirement requires that the region of interest is within a predetermined region of the area..

11. The method of any preceding claim, wherein monitoring the infant comprises at least one of:
monitoring one or more of the infant's vital signs;
monitoring the infant's movement;
monitoring the infant's position; and
tracking the infant's sleep stages.

12. A computer program comprising code means for implementing the method of any preceding claim when said program is run on a processing system.

13. A processing arrangement comprising the computer program of claim 12 and configured to execute the computer program.

14. A system for determining a region of interest of an area for use in monitoring an infant within the area, the system comprising:
a processing arrangement according to claim 13.

15. An infant monitoring system, comprising:
the system of claim 14; and
an image sensor configured to capture image data of the infant in the area.
